# EUROPÄISCHE PATENTSCHRIFT

(11) **EP 1 587 497 B1**
(45) Veröffentlichungstag und Bekanntmachung des Hinweises auf die Patenterteilung: **12.11.2014**
(21) Anmeldenummer: 03767591.5
(22) Anmeldetag: 21.11.2003
(51) Int. Cl.: A61K 9/00, A61J 3/10, A61K 9/16

(54) **VERFAHREN ZUR HERSTELLUNG EINER ORALEN ARZNEIFORM MIT UNMITTELBAREM ZERFALL UND WIRKSTOFFFREISETZUNG**
METHOD FOR PRODUCING AN IMMEDIATELY DECOMPOSING ORAL FORM OF ADMINISTRATION WHICH RELEASES ACTIVE INGREDIENTS
PROCEDE DE PRODUCTION D'UNE FORME PHARMACEUTIQUE ORALE A DECOMPOSITION IMMEDIATE ET LIBERATION DE PRINCIPES ACTIFS

(30) Priorität: 28.01.2003 DE 10304403
(43) Veröffentlichungstag der Anmeldung: 26.10.2005
(73) Patentinhaber: Evonik Röhm GmbH, 64293 Darmstadt (DE)
(72) Erfinder: PETEREIT, Hans-Ulrich, 64291 Darmstadt (DE); MEIER, Christian, 64295 Darmstadt (DE); GRYCZKE, Andreas, 64347 Griesheim (DE)
(86) Internationale Anmeldenummer: PCT/EP2003/013059
(87) Internationale Veröffentlichungsnummer: WO 2004/066976

(56) Entgegenhaltungen:
- EP-A- 0 417 588
- WO-A-03/007917
- US-A1- 2002 168 404

## Beschreibung

### Gebiet der Erfindung

Die Erfindung betrifft ein Verfahren zur Herstellung einer oralen Arzneiform mit unmittelbarem Zerfall und Wirkstofffreisetzung bereits im Mund. Die Erfindung betrifft weiterhin ein wirkstoffhaltiges Pulvers und dessen Verwendung.

### Stand der Technik

EP-A 0 417 588 beschreibt ein Verfahren zur Herstellung eines komplexierten Arzneimittels aus einem ionogenen Wirkstoff durch Umsetzung des Wirkstoffs mit einem komplementär ionogenen, partikelförmigen Polymer in Gegenwart einer zum Anfeuchten der Mischung ausreichenden Wassermenge. Bei Wirkstoffssalzen ist es erforderlich, der Mischung eine Säure oder Base zur Neutralisation des Gegenions des Wirkstoffs zuzusetzen. Bei der Umsetzung von Wirkstoffsalzen wie Propranolol-HCl, Verapamil-HCl oder Metoclopramid-HCl mit anionischen (Meth)acrylat-Copolymeren wie EUDRAGIT® L oder EUDRAGIT® L100-55 wird der Mischung beispielsweise Natriumcarbonat zugesetzt. In diesem Fall kann eine Geschmacksisolierung der bitter schmeckenden Wirkstoffe erreicht werden. Weiterhin ist erwähnt, daß im Falle, daß der ionogene Wirkstoff eine Säure ist, ein partikelförmiges Polymer eingesetzt werden kann, welches als komplementär ionogene Gruppen seitenständige Aminogruppen aufweist. Die seitenständige Aminogruppen kann z. B. eine tertiäre Aminogruppe, stammend aus polymerisierten Monomeren wie z. B. 2-Dimethylamino-ethyl-methacrylat sein.

WO 01/39751 beschreibt ein Verfahren zur Herstellung von Formkörpern mittels Spritzguß mit den Verfahrensschritten
a) Aufschmelzen eines (Meth)acrylat-Copolymeren, das sich aus 30 bis 80 Gew.-% radikalisch polymerisierten C1- bis C4-Alkylestern der Acryl- oder der Methacrylsäure und 70 bis 20 Gew.-% (Meth)acrylat-Monomeren mit einer tertiären Ammonium- bzw. Aminogruppe im Alkylrest zusammensetzt,
   wobei das (Meth)acrylat-Copolymere in Mischung mit 1 bis 70 Gew.-% von einem Weichmacher und einem Trockenstellmittel im Verhältnis 1: 1 bis 1 : 20 vorliegt,
   wobei mindestens 1 Gew.-% Weichmacher enthalten ist,
   sowie 0,05 bis 5 Gew.-% eines Trennmittels enthalten sind und
   zusätzlich weitere übliche Additive oder Hilfsstoffe und gegebenenfalls ein pharmazeutischer Wirkstoff in der Mischung enthalten sein können und die Mischung vor dem Aufschmelzen einen Gehalt an niedrigsiedenden Bestandteilen mit einem Dampfdruck von mindestens 1,9 bar bei 120°C von über 0,5 Gew.-% aufweist,
b) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
c) Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

WO 02/67906 beschreibt ein Verfahren zur Herstellung eines Überzugs- und Bindemittels für orale oder dermale Arzneiformen bestehend im wesentlichen aus (a) einem Copolymer, bestehend aus radikalisch polymerisierten C1- bis C4-Estern der Acryl- oder Methacrylsäure und weiteren (Meth)acrylatMonomeren die funktionelle tertiäre Ammoniumgruppen aufweisen, wobei das Copolymer in Pulverform mit einer mittleren Teilchengröße von 1 - 40 µm vorliegt, (b) 3 bis 15 Gew.-%, bezogen auf (a), eines Emulgators mit einem HLB-Wert von mindestens 14 und (c) 5 bis 50 Gew.-%, bezogen auf (a), einer C₁₂- bis C₁₈-Monocarbonsäure oder einer C₁₂- bis C₁₈-Hydroxylverbindung, wobei die Komponenten (a), (b) und (c) mit oder ohne Zusatz von Wasser und gegebenenfalls unter Zusatz eines pharmazeutischen Wirkstoffs und weiterer üblicher Zuschlagstoffe miteinander vermengt oder vermischt werden und das Überzugs- und Bindemittel aus der Mischung durch Schmelzen, Gießen, Ausstreichen, Aufsprühen oder Granulieren hergestellt wird.

Gemäß der WO 02/67906 sind besonders lagerstabile Arzneiformen erhältlich, die insbesondere feuchteempfindliche Wirkstoffe wie Acetylsalicyläure, Carbenoxolon, Cefalotin, Epinefrin, Imipramin, Kaliumjodid, Ketoprofen, Levodopa, Nitrazepam, Nitroprussid, Oxitetracyclin-HCl, Promethazin, Omeprazol oder andere Benzimidazolderivate oder Streptomycin enthalten können.

Wirkstoffklassen und Substanzen, die oftmals bitteren Geschmack hervorrufen können und sich mit den Überzugs- und Bindemittel gemäß der WO 02/67906 vorteilhafterweise auch geschmacksisolierend formulieren lassen sind z. B.:
Analgetika und Antirheumatika: Paracetamol, Diclofenac, Aceclofenac, Ibuprofen, Ketoprofen, Flubiprofen, Levacetylmethadol, Oxycodon
Psychopharmaka: Prometazine, Donepezil, Modafinil, Nefazodon, Reboxetin, Sertindol, Sertralin
Antibiotika: Erythromicyn, Roxithromycin, Clarithromycin, Grepafloxacin,
Ciprofloxacin, Levofloxacin, Sparfloxacin, Trovafloxacin, Nevirapin
Betablocker: Propanolol, Metoprolol, Bisoprolol, Nebivolol
Antidiabetika: Metformin, Miglitol, Repaglinid
H1 Antihistaminika: Diphenhydramin, Fexofenadin, Mizolastin
H2 Antihistaminika: Cimetidin, Nizatidin, Ticlopidin, Cetridin, Ranitidin,
Vitamine: Thiaminenitrate;
   sowie weitere Wirkstoffe: Chinidin-Sulfat, Amiloprilose-HCl, Pseudoephedrin -HCl, Sildenafil, Topiramat, Granisetron, Rebamipide, Chinin-HCl

WO03/007917A1 beschreibt multipartikuläre Tabletten, enthaltend ein Protonenpumpen inhibierendes Agens, einen "antacid"-Bestandteil sowie weitere Hilfsstoffe, wie z. B. EUDRAGIT® E PO. US2002/168404A1 beschreibt die Verwendung eines (Meth)acrylat-Copolymeren vom Typ C nach USPINF, z. B. EUDRAGIT®100-55, als Sprengmittel oder Hilfsstoff in einer Tablette.

### Aufgabe und Lösung

Ein Problem bei vielen oralen Arzneiformen, ist daß das Herunterschlucken oftmals die Zuhilfenahme von Flüssigkeit, z. B. einem Schluck Wasser, erfordert. Dies ist ungünstig, wenn im Bedarfsfall kein Getränk zur Verfügung steht oder etwa die momentane berufliche Tätigkeit unterbrochen werden muß, um das Medikament einnehmen zu können. Für viele Patienten ist es zudem unangenehm in Gegenwart anderer Personen quasi beobachtet und Aufmerksamkeit erregend ihr Medikament einzunehmen, was umso auffälliger ist, wenn nach einem Getränk benutzt werden muß oder für diesen Zweck gar erbeten werden muß.

Viele Patienten, insbesondere zu nennen ältere Menschen und Kinder, wünschen daher orale Arzneiformen, die einfach und unauffällig praktisch an beliebigen Orten eingenommen werden können. Dies ist insbesondere bei Krankheiten der Fall, die sehr pünktlich oder bei Bedarf unverzüglich eingenommen werden sollen oder müssen, wie z. B. bei Schmerzmitteln.

Es besteht zusätzlich ein Bedarf an Arzneiformen, die den enthalten Wirkstoff z. B. Schmerzmittel bei oraler Einnahme bereits im Mund freisetzen und auf diese Weise rasch wirken können. Bekannte Applikationsformen sind z. B. verpreßten Tabletten oder Lutschtabletten, gefriergetrockneten Tabletten, gegossenen Tabletten oder Pastillen, Sachets, Kautabletten, Trockensäften und/oder flüssigkeitsgefüllten Bonbons.

Viele dieser schnell zerfallenden Arzneiformen haben jedoch den Nachteil, daß sie einen sandigen Mundgeschmack bewirken, der einige Minuten andauern kann, bis sich die Tablettenbestanteile völlig aufgelöst haben. Das sandige Mundgeschmack wird als unangenehm empfunden wird und kann einen Hustenreiz bewirken. Ein weiteres Problem ist dabei die Geschmacksisolierung von bitter schmeckenden Wirkstoffen. Wegen der Anforderung der Wirkstoffreisetzung im Mund können die bekannten geschmacksisolierenden Überzüge nicht verwendet werden.

Zur Lösung dieser Probleme sollte eine Arzneiform bereitgestellt werden, die ohne Flüssigkeit einnehmbar ist und den Wirkstoff ummittelbar freisetzt. Dabei soll ein sandiger Mundgeschmack ausbleiben. Die Arzneiform soll für eine Vielzahl von Wirkstoffen, insbesondere jedoch für Schmerzmittel der Klasse der Antirheumatika oder für Antibiotika geeignet sein.

Die Aufgabe wird gelöst durch ein Verfahren zur Herstellung einer oralen Arzneiform mit unmittelbarem Zerfall und Wirkstofffreisetzung bereits im Mund, durch intensives Mischen
(a) eines anionischen pharmazeutischen Wirkstoffs mit
(b) einem Copolymer, bestehend aus radikalisch polymerisierten C₁- bis C₄-Estern der Acryl- oder Methacrylsäure und weiteren (Meth)acrylat-Monomeren die funktionelle tertiäre Aminogruppen aufweisen, sowie
(c) 5 bis 50 Gew.-%, bezogen auf (b), einer C₁₂- bis C₂₂-Carbonsäure
in der Schmelze, Erstarren der Mischung und Mahlen zum einem wirkstoffhaltigen Pulver mit einer mittleren Korngröße von 200 µm oder weniger, Einbetten des Pulvers in eine wasserlösliche Matrix aus pharmazeutisch üblichen Hilfsstoffen, mit der Maßgabe, daß nicht mehr als 3 Gew.-%, bezogen auf das Copolymer, an Emulgatoren mit einem HLB-Wert von mindestens 14 enthalten sein dürfen.

In bisher nicht verstandener Weise ergeben sich die Vorteile der Erfindung anders als bei der WO 02/67906 nur bei anionischen Wirkstoffen. Möglicherweise ergibt sich eine thermisch induzierte Wechselwirkung der anspruchsgemäßen Bestandteile (a), (b) und (c), die in dieser Weise nicht aus der WO 02/67906 ableitbar ist. Die erfindungsgemäß erhältlichen Arzneiformen sind gut ohne zusätzliche Flüssigkeit einnehmbar und verursachen nach Wirkstofffreisetzung im Mund keinen sandigen Geschmack.

### Ausführung der Erfindung

Die Erfindung betrifft ein Verfahren zur Herstellung einer oralen Arzneiform mit unmittelbarem Zerfall und Wirkstofffreisetzung bereits im Mund, durch intensives Mischen
(a) eines anionischen pharmazeutischen Wirkstoffs mit
(b) einem Copolymer, bestehend aus radikalisch polymerisierten C₁- bis C₄-Estern der Acryl- oder Methacrylsäure und weiteren (Meth)acrylatMonomeren die funktionelle tertiäre Aminogruppen aufweisen, sowie
(c) 5 bis 50 Gew.-%, bezogen auf (b), einer C₁₂- bis C₂₂-Carbonsäure

in der Schmelze, Erstarren der Mischung und Mahlen zum einem wirkstoffhaltigen Pulver mit einer mittleren Korngröße von 200 µm oder weniger, Einbetten des Pulvers in eine wasserlösliche Matrix aus pharmazeutisch üblichen Hilfsstoffen, mit der Maßgabe, daß nicht mehr als 3 Gew.-%, bezogen auf das Copolymer, an Emulgatoren mit einem HLB-Wert von mindestens 14 enthalten sein dürfen.

### Pharmazeutischer Wirkstoff (a)

Der anionische pharmazeutischen Wirkstoff, liegt bedingt durch die Herstellung in der Schmelze eingebettet im Copolymer in Form einer "solid solution" vor. Der Zustand der "solid solution" kann z. B. im Polarisationsmikroskop, thermoanalytisch (Differential Scanning Calorimetry (DSC)) oder im Röntgenbeugungsspektrum nachgewiesen werden.

Das Mengenverhältnis bezogen auf Gew.-% von Wirkstoff zu Copolymer liegt günstigerweise bei 2 zu 1 bis 1 zu 2. Bevorzugt ist das Copolymer in gleichen Mengen oder im Überschuß vorhanden.

Der anionischer Wirkstoff (a) ist bevorzugt ein anionisches Schmerzmittel ein anionisches Antirheumatikum oder ein anionisches Antibiotikum.

Das wirkstoffhaltige Pulver kann z. B. die folgenden anionischer Wirkstoff enthalten:
Acamprosat, Aceclofenac, Acemetacin, Acetylcystein, Acetylsalicylsäure, Acetyltyrosin, Acipimox, Acitretin , Alanin, Alendronsäure, Amethopterin, Aminosäuren, Amoxicillin, Ampicillin , Ascorbinsäure, Atorvastatin , Azidocillin, Aztreonam, Bacampicillin, Baclofen, Benazepril, Bendamustin, Benzylpenicillin, Bezafibrat, Biotin, Bornaprin, Bumetanid, Cabastin, Canrenoinsäure, Carbamoylphenoxyessigsäure, Carbidopa, Carbimazol, Carbocistein, Carisoprodol, Cefaclor, Cefadroxil, Cefalexin, Cefazolin, Cefepim, Cefetamet, Cefixim, Cefotaxim, Cefotiam, Cefoxitin, Cefpodoxim, Ceftazidim, Ceftibuten , Ceftriaxon, Cefuroxim, Cetirizin, Chenodeoxycholsäure, Chlorambucil, Cidofovir, Cilastatin, Cilazapril, Cinoxacin, Ciprofloxacin, Cisatracurium besilat, Clavulansäure, Clodronsäure, Clorazepat, Cromoglicinsäure, Desmeninol,
Diclofenac, Dicloxacillin, Enoxacin, Eprosartan, Etacrynsäure, Etidronsäure, Etofyllin, Etomidat, Felbinac, Felodipin, Fenofibrat, Fexofenadin, Flavoxat, Fleroxacin, Flucloxacillin, Flufenaminsäure, Flumazenil, Flupirtin, Flurbiprofen, Fluvastatin, Fosfomycin, Fosinopril, Furosemid, Fusidinsäure, Gabapentin, Gemfibrozil, Ibandronsäure, Ibuprofen, Iloprost, Imidapril, Imipenem, Indomethacin, Irinotecan, Isradipin, Ketoprofen, Lercanidipin , Levodopa, Levofloxacin, Liothyronin, Liponsäure, Lisinopril, Lodoxamid, Lomefloxacin, Lonazolac, Loracarbef, Loratadin, Lovastatin, Mefenaminsäure, Meropenem, Mesalazin, Metamizol, Methotrexat, Methyldopa, Mezlocillin, Moexipril, Montelukast, Moxifloxacin, Mupirocin, Naproxen, Natamycin, Nateglinid, Nedocromil, Nicotinsäure, Nifedipin, Nilvadipin, Nimodipin, Nisoldipin, Nitrendipin, Norfloxacin, Ofloxacin, Olsalazin, Orotsäure, Oxacillin, Pamidronsäure, Pangamsäure, Penicillamin, Phenoxymethylpenicillin, Pentosanpolysulfat, Perindopril, Pethidin, Pipemidsäure, Piperacillin, Pirenoxin, Piretanid, Probenecid, Proglumid, Propicillin, Prostaglandine, Quinapril, Quinaprilat, Ramipril, Repaglinid, Reserpin, Risedronsäure, Salicylsäure, Sulfasalazin, Spirapril, Sulbactam, Sulfasalazin, Sultamicillin, Tazaroten, Tazobactam, Telmisartan, Tiagabin, Tiaprofensäure, Tilidin, Tiludronsäure, Trandolapril, Tranexamsäure, Valproinsäure Vigabatrin, Vincamin, Vinpocetin, Zanamivir, Zoledronsäure, Zopiclon und/oder deren Salze, Isomere und/oder Kombinationen enthalten sind.

### Copolymer (b)

Die Copolymere (a) bestehen im wesentlichen oder ganz aus radikalisch polymerisierten C1- bis C4-Estern der Acryl- oder Methacrylsäure und weiteren (Meth)acrylat-Monomeren, die funktionelle tertiäre Aminogruppen aufweisen. Geeignete Monomere mit funktionellen tertiären Aminogruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären Aminogruppen im Copolymeren kann vorteilhafterweise zwischen 30 und 70 Gew.-%, bevorzugt zwischen 40 und 60 Gew.-% liegen. Der Anteile der C1- bis C4-Estern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat, Butylacrylat.

Ein der Komponente (b) entsprechendes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein. Der Anteil der Komponente (a) an der Formulierung beträgt bevorzugt 50 - 90 Gew.-%.

Die Copolymere (b) werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in geeignete Teilchengrößenbereich gebracht werden. Geeignet sind Granulate und Pulver. Geeignete Handelsprodukte sind z. B. EUDRAGIT® E 100 (Granulat) oder EUDRAGIT® E PO (Pulver).

### Komponente (c)

Komponente (c): 5 bis 50, bevorzugt 10 bis 20 Gew.-% (bezogen auf die Copolymer-Komponente (b) einer C₁₂- bis C₂₂-Carbonsäure. Die Komponente (c) ist wichtig für die Verarbeitbarkeit. Bevorzugt sind unverzweigte C₁₂- bis C₂₂-Monocarbonsäuren. Es können gegebenenfalls auch verzweigte Derivate der genannten Substanzen geeignet sein.

C₁₂- bis C₂₂-Monocarbonsäuren sind z.B insbesondere Laurinsäure und Myristinsäure. Bevorzugt sind Palmitinsäure und Stearinsäure.

### Emulgatoren mit einem HLB-Wert von mindestens 14

Emulgatoren mit einem HLB-Wert von mindestens 14 sollen zu weniger als 3 Gew.-%, bevorzugt weniger als 2 oder 1 Gew.-%, insbesondere soll kein solcher Emulgator enthalten sein. Der Grund dafür liegt in der Pulverstruktur des Ausgangsmaterials, in dem der Eigengeschmack solcher Emulgatoren besonders hervortritt. Überraschenderweise ist im Gegensatz zur Lehre der WO 02/67906 unter Anwendung des speziellen erfindungsgemäßen Verfahrens ein Verzicht auf den Emulgatoreinsatz möglich.

Emulgatoren oder Tenside sind grenzflächenaktive Substanzen mit lyobipolarem Charakter, d.h. in ihrem Molekül müssen unpolare, lipophile und polare, hydrophile Zentren vorliegen (P.H. List, Arzneiformenlehre, Wissenschaftliche Verlagsgesellschaft mbH Stuttgart,1982,Kap. 6.2.). Je nach molekularem Aufbau unterscheidet man zwischen ionogenen und nichtionogen Emulgatoren.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 14.

Unter Emulgatoren mit einem HLB-Wert über 14 werden hydrophile, nicht ionische Emulgatoren mit HLB - Bereich von mindestens 14 sowie ebenfalls hydrophile, anionische Emulgatoren und deren Salze, die einen rechnerischen HLB-Wert über 14 aufweisen, verstanden. Beispiele für Emulgatoren mit einem HLB-Wert über 14 sind z. B. Natriumlaurylsulfat und Natriumcetylstearylsulfat, Saccharosestearat und Polysorbat 80.

Emulgatoren mit HLB-Werten von weniger als 14, wie z. B. Glycerolmonostearat können hingegen auch in Mengen von mehr als 3 Gew.-% enthalten sein.

### Pharmazeutisch übliche Hilfsstoffe

Das Pulvers wird in eine wasserlösliche Matrix aus pharmazeutisch üblichen Hilfsstoffen eingebettet.

### Füll- und Bindemittel

Die wasserlösliche Matrix wird überwiegend gebildet aus Füll- und Bindemitteln. Bevorzugt sind dies z. B. wasserlösliche Mono-, Di, Oligo- oder Polysaccharide oder deren Derivate, weiterhin Peptide, Proteine etc.. Beispiele sind z. B. Lactose, Fructose, Glucose, Dextrose, Galaktose, Mannit, Rhamnose, Tragant, Dextrin, Guar Gum, Sorbitol, Xylitol, Isomatose, Saccharose, Maltose, Hydroxypropylmethylcellulose (HPMC), Stärkehydrolysate, Gelatine.

Einsatzmengen und Verwendung der üblichen Zuschlagstoffe in Arzneimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften.

### Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca-Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,1 bis 10 Gew.-% bezogen auf die Arzneiform.

### Pigmente:

Der Zusatz erfolgt nur selten in Form des löslichen Farbstoffs. In der Regel dispergiert man Aluminium- oder Eisenoxidpigmente. Übliche Einsatzmengen für Pigmente in den erfindungsgemäßen Überzugs- und Bindemitteln zwischen 1 und 10 Gew.-%, bezogen auf die Arzneiform.

Grundsätzlich müssen natürlich alle eingesetzten Substanzen toxikologisch unbedenklich und in Arzneimitteln ohne Risiko für Patienten zu verwenden sein.

Weitere Zuschlagstoffe können auch Weichmacher sein. Übliche Mengen liegen zwischen 0 und 50, bevorzugt 0 bis 20, insbesondere 0 bis 10 Gew.-%. Besonders bevorzugt sind allerdings höchstens 5 Gew.-% oder kein Weichmacher enthalten, da die Formulierungen durch die Anwesenheit der Komponenten (c) häufig bereits elastisch genug sind und zusätzlicher Weichmacher zu unerwünschter Klebrigkeit führen kann.

### Weichmacher:

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet. Auch können Mischungen von Weichmachern eingesetzt werden.

### Das Herstellungsverfahren

Das erfindungsgemäße Verfahren sieht die Herstellung einer oralen Arzneiform mit unmittelbarem Zerfall.und Wirkstofffreisetzung bereits im Mund, durch intensives Mischen der Komponenten (a), (b) und (c) im der Schmelze vor. Geeignet sind Verarbeitungstemperaturen im Bereich von 80 bis 200 °C, bevorzugt von 100 bis 180 °C. Bevorzugt setzt man zum Zweck des intensiven Mischens in der Schmelze einen Doppelschneckenextruder ein. Nach dem Erstarren wird die Mischung zum einem wirkstoffhaltigen Pulver gemahlen. Die mittleren Korngröße des Pulvers soll 200 µm oder weniger, bevorzugt 50 bis 150 µm betragen.

Die mittlere Korn- bzw. Teilchengröße der Pulver kann wie folgt bestimmt werden: Durch Luftstrahlsiebung zur einfachen Aufteilung des Mahlproduktes in wenige Fraktionen. Diese Methode ist in diesem Meßbereich etwas ungenauer als die Alternativen. Mindestens 70, bevorzugt 90 % der Teilchen bezogen auf die Masse (Masseverteilung) sollen jedoch in dem erfindungsgemäßen Größenbereich von 200 µm oder weniger, bevorzugt von 50 bis 150 µm liegen. Eine gut geeignete Meßmethode ist die Laserbeugung zur Bestimmung der Korngrößenverteilung. Handelsübliche Geräte erlauben die Messung in Luft (Fa. Malvern S3.01 Partikelsizer) oder bevorzugt in flüssigen Medien (Fa. LOT, Galai CIS 1). Voraussetzung für die Messung in Flüssigkeiten ist, das sich das Polymer darin nicht löst oder die Teilchen auf eine andere Weise während der Messung verändern. Ein geeignetes Medium ist z. B. eine stark verdünnte (ca. 0,02%ige) wäßrige Polysorbat 80 Lösung.

Das wirkstoffhaltige Pulver kann zu einer Tablette, Lutschtablette, gefriergetrockneten Tabletten, gegossenen Tabletten oder Pastillen, Sachets, Kautabletten, Trockensäften, Bonbons und/oder flüssigkeitsgefüllten Bonbons verarbeitet werden.

Diese Verarbeitung erfolgt in der Regel in mehreren Schritten. Zunächst wird das wirkstoffhaltige Copolymerpulver mit pharmazeutischen Hilfsstoffen gemischt und kann z. B. direkt zu Tabletten, Lutschtabletten oder Kautabletten verpreßt werden. Das Gemisch kann auch mit Wasser angeteigt, in eine Form gefüllt und gefriergetrocknet werden, so daß man gefriergetrocknete Tabletten erhält. Gegossene Tabletten oder Pastillen können erhalten werden, indem man das wirkstoffhaltige Copolymerpulver, z. B. mit einer Saccharidlösung bei erhöhter Temperatur mischt, in eine Form, Z. B. für Tabletten oder Bonbons gießt und durch Abkühlen erstarren läßt. Flüssigkeitsgefüllte Bonbons können erzeugt werden, indem man ein flüssiges Gemisch, enthaltend das wirkstoffhaltige Copolymerpulver z. B. in einer Zuckerlösung in eine feste Hülle aus z. B. einem Zucker einspritzt und diese anschließend verschließt.

### Wirkstoffhaltiges Pulver

Das wirkstoffhaltige Pulver hat eine mittlere Korngröße von 200 µm oder darunter, bevorzugt 50 bis 150 µm und enthält
(a) einen anionischen pharmazeutischen Wirkstoff, der in Form einer solid solution vorliegt und eingebettet ist in
(b) ein Copolymer, welches aus radikalisch polymerisierten C₁- bis C₄-Estern der Acryl- oder Methacrylsäure und weiteren (Meth)acrylat-Monomeren die funktionelle tertiäre Aminogruppen aufweisen, besteht, sowie
(c) 5 bis 50 Gew.-%, bezogen auf (b), einer C₁₂- bis C₂₂-Carbonsäure,
(d) mit der Maßgabe, daß kein oder weniger als 3 Gew.-%, bezogen auf das Copolymer, eines Emulgators mit einem HLB-Wert von mindestens 14 enthalten ist.

### Verwendungen

Das wirkstoffhaltiges Pulvers kann zur Herstellung einer oralen Arzneiform mit unmittelbarem Zerfall und Wirkstofffreisetzung bereits im Mund, die nach Freisetzung für mindestens 30 Sekunden keinen bitteren Geschmack hervorruft, verwendet werden. Die Arzneiform kann in Form von verpreßten Tabletten oder Lutschtabletten, gefriergetrockneten Tabletten, gegossenen Tabletten oder Pastillen, Sachets, Kautabletten, Trockensäften, Bonbons und/oder flüssigkeitsgefüllten Bonbons vorliegen.

### Bitterwerte

Die Überprüfung der Geschmacksisolierung kann auf einfache Weise organoleptisch durch Verkosten erfolgen. Bei dieser Prüfung soll nach Wirkstoffreisetzung für mindestens 30 Sekunden noch kein oder leicht bitterer Geschmack wahrnehmbar sein. Genauer ist die Bestimmung von Bitterwerten. Kein oder leicht bitterer Geschmack entspricht Bitterwerten unter 1000. Bitterwerte können nach DAB 1999 Methode 2.8.N8 (Bestimmung des Bitterwertes) bestimmt werden.

Während z. B. Ibuprofen einen Bitterwert um die 100.000 hat, liegt der erfindungsgemäße Wert für einen eingebetteten anionischen Wirkstoff in der Regel unter 1000, bevorzugt unter 100. Ein Bitterwert von 1000 ist für pharmazeutische Praxis in der Regel ausreichend.

### BEISPIELE

In den Beispielen verwendete Copolymere:
EUDRAGIT® E PO: Copolymerpulver aus Methylmethacrylat, Butylmethacrylat,
und Dimethylaminoethylmethacrylat in Verhältnis 25 : 25 : 50 mit einer mittleren Teilchengröße von 15 µm.

EUDRAGIT® E 100 : Copolymer aus Methylmethacrylat, Butylmethacrylat, und Dimethylaminoethylmethacrylat in Verhältnis 25 : 25 : 50 im Granulatform.

Die Wirksamkeit der Geschmacksisolierung wurde organoleptisch durch Verkosten geprüft. Dabei wurde die Zeit zwischen Aufnahme in den Mund und Auftreten des bitteren Geschmacks ermittelt.

### Beispiel 1:

Compound mit 1 mol Dimethylaminoethylmethacrylat-Einheiten enthalten im Copolymer EUDRAGIT® E PO: 1 mol Stearinsäure : 0,66 mol Ibuprofen : 0,18 mol Talk.

Es wurden 39,42 g EUDRAGIT^{®} E PO, 35,2 g Stearinsäure, 16,9 g Ibuprofen und 8,4 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 2 min nicht bitter.

### Beispiel 2:

Compound mit 1 mol Dimethylaminoethylmethacrylat-Einheiten enthalten im Copolymer EUDRAGIT® E PO : 0,5 mol Stearinsäure : 0,66 mol Ibuprofen : 0,18 mol Talk.

Es wurden 47,85 g EUDRAGIT^{®} E PO, 21,38 g Stearinsäure, 20,5 g Ibuprofen und 10,25 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 2 min nicht bitter bis leicht bitter.

### Beispiel 3:

Compound mit 1 mol Dimethylaminoethylmethacrylat-Einheiten enthalten im Copolymer EUDRAGIT® E PO : 0,65 mol Stearinsäure : 0,65 mol Ibuprofen : 0,18 mol Talk

Es wurden 44,8 g EUDRAGIT^{®} E PO, 26,4 g Stearinsäure, 19,2 g Ibuprofen und 9,6 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 2 min nicht bitter.

### Beispiel 4:

Compound mit 1 mol Dimethylaminoethylmethacrylat-Einheiten enthalten im Copolymer EUDRAGIT® E PO: 0,33 mol Stearinsäure : 0,66 mol Ibuprofen : 0,18 mol Talk

Es wurden 51,6 g EUDRAGIT^{®} E PO, 15,23 g Stearinsäure, 22,1 g Ibuprofen und 11 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 1 min leicht bitter.

### Beispiel 5:

Compound mit 1 mol Dimethylaminoethylmethacrylat-Einheiten enthalten im Copolymer EUDRAGIT® E PO: 0,34 mol Stearinsäure : 1 mol Ibuprofen : 0,27 mol Talk.

Es wurden 34,73 g EUDRAGIT^{®} E PO, 15,52 g Stearinsäure, 33,1 g Ibuprofen und 16,58 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 1 min bitter.

### Vergleichsbeispiel 6: (Wirkstoff Coffein nicht erfindungsgemäß)

Compound mit 1-mol Dimethylaminoethylmethacrylat-Einheiten enthalten im Copolymer EUDRAGIT® E PO : 0,5 mol Stearinsäure : 1,58 mol Coffein (Fₚ: 234 - 239 °C).

Es wurden 41,47 g EUDRAGIT^{®} E PO, 18,53 g Stearinsäure, 40 g Coffein eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 10 s bitter.

### Vergleichsbeispiel 7: (ohne Stearinsäure)

Compound mit 1 mol Dimethylaminoethylmethacrylat-Einheiten enthalten im Copolymer EUDRAGIT® E PO: 0,67 mol Ibuprofen : 0,18 mol Talk.

Es wurden 60 g EUDRAGIT^{®} E PO, 26,4 g Ibuprofen und 13,2 g Talk eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.
Nimmt man 1 g dieses Compounds in den Mund, schmeckt er nach 10 s bitter.

### Vergleichsbeispiel 8: (Wirkstoff Paracetamol nicht erfindungsgemäß)

Compound mit 1 mol Dimethylaminoethylmethacrylat-Einheiten enthalten im Copolymer EUDRAGIT® E PO: 0,5 mol Stearinsäure : 2,03 mol Paracetamol (Fp: 168 - 172 °C).

Es wurden 41,47 g EUDRAGIT^{®} E PO, 18,53 g Stearinsäure, 40 g Paracetamol eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt Nimmt man 1 g dieses Compounds in den Mund, schmeckt er sofort bitter.

### Vergleichsbeispiel 9: (Wirkstoff Paracetamol nicht erfindungsgemäß)

Compound mit 1 mol Dimethylaminoethylmethacrylat-Einheiten enthalten im Copolymer EUDRAGIT® E : 0,5 mol Stearinsäure : 1 mol Paracetamol.

Es wurden 41,5 % EUDRAGIT^{®} E 100, 18,53 % Stearinsäure und 40 % Paracetamol zusammen in einem 18 mm Doppelschneckenextruder in einem Temperaturbereich von 100 °C bis 172 °C extrudiert. In dem Bereich des Extruders, wo 172 °C bestanden, war die Schnecke besonders mischintensiv ausgelegt um eine homogene Schmelze zu erreichen.

Der so entstandene Compound schmeckt sofort bitter.

### Beispiel 10:

Compound mit 1 mol Dimethylaminoethylmethacrylat-Einheiten enthalten im Copolymer EUDRAGIT® E PO: 0,06 mol Stearinsäure : 0,77 mol Ibuprofen.

Es wurden 100 g EUDRAGIT^{®} E PO, 5 g Stearinsäure und 50 g Ibuprofen eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 30 - 60 s min bitter.

### Beispiel 11:

Compound mit 1 mol Dimethylaminoethylmethacrylat-Einheiten enthalten im Copolymer EUDRAGIT® E PO: 0,12 mol Stearinsäure : 0,77 mol Ibuprofen. Es wurden 100 g EUDRAGIT^{®} E PO, 10 g Stearinsäure und 50 g Ibuprofen eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 1 min bitter.

### Vergleichsbeispiel 12 (C₁₂-Alkohol-Verbindung anstelle von Stearinsäure)

Compound mit 1 mol Dimethylaminoethylmethacrylat-Einheiten enthalten im Copolymer EUDRAGIT® E PO: 0,34 mol Dodecanol : 0,77 mol Ibuprofen.

Es wurden 100 g EUDRAGIT^{®} E PO, 20 g Dodecanol und 50 g Ibuprofen eingewogen und zusammen in den auf 100 °C vorgewärmten IKA Messkneter gegeben, wo die Mischung bei 100 °C Produkttemperatur für 20 min mit 60 U/min (2 Knetschaufeln) geknetet wurde. Die Mischung wurde dem Messkneter entnommen und mit Trockeneis abgekühlt.

Nimmt man 1 g dieses Compounds in den Mund, schmeckt er nach 20 s bitter und weist den unangenehmen Geschmack von Dodecanol auf.

## Patentansprüche

1. Verfahren zur Herstellung einer oralen Arzneiform mit unmittelbarem Zerfall und Wirkstofffreisetzung bereits im Mund, durch intensives Mischen
(a) eines anionischen pharmazeutischen Wirkstoffs mit
(b) einem Copolymer, bestehend aus radikalisch polymerisierten C₁- bis C₄-Estern der Acryl- oder Methacrylsäure und weiteren (Meth)acrylatMonomeren die funktionelle tertiäre Aminogruppen aufweisen, sowie
(c) 5 bis 50 Gew.-%, bezogen auf (b), einer C₁₂- bis C₂₂-Carbonsäure
in der Schmelze, Erstarren der Mischung und Mahlen zum einem wirkstoffhaltigen Pulver mit einer mittleren Korngröße von 200 µm oder weniger, Einbetten des Pulvers in eine wasserlösliche Matrix aus pharmazeutisch üblichen Hilfsstoffen, mit der Maßgabe, daß nicht mehr als 3 Gew.-%, bezogen auf das Copolymer, an Emulgatoren mit einem HLB-Wert von mindestens 14 enthalten sein dürfen.

2. Verfahren nach Anspruch 1, **dadurch gekennzeichnet, daß** man zum Zweck des intensiven Mischens in der Schmelze einen Doppelschneckenextruder einsetzt.

3. Verfahren nach Anspruch 1 oder 2, **dadurch gekennzeichnet, daß** man bei Temperaturen im Bereich von 80 bis 200 °C extrudiert.

4. Verfahren nach einem oder mehreren der Ansprüche 1 bis 3, **dadurch gekennzeichnet, daß** das Einbetten des Pulvers in die wasserlösliche Matrix durch Verpressen, Gießen, Granulieren oder Gefriertrocknen erfolgt.

5. Wirkstoffhaltiges Pulver mit einer mittleren Korngröße von 200 µm oder darunter, enthaltend
(a) einen anionischen pharmazeutischen Wirkstoff, welcher in Form einer solid solution vorliegt und eingebettet ist in
(b) ein Copolymer, welches aus radikalisch polymerisierten C₁- bis C₄-Estern der Acryl- oder Methacrylsäure und weiteren (Meth)acrylatMonomeren die funktionelle tertiäre Aminogruppen aufweisen, besteht, sowie
(c) 5 bis 50 Gew.-%, bezogen auf (b), einer C₁₂- bis C₂₂-Carbonsäure,
(d) mit der Maßgabe, daß kein oder weniger als 3 Gew.-%, bezogen auf das Copolymer, eines Emulgators mit einem HLB-Wert von mindestens 14 enthalten ist.

6. Wirkstoffhaltiges Pulver nach Anspruch 5, **dadurch gekennzeichnet, daß** als anionischer Wirkstoff (a) ein anionisches Schmerzmittel bzw. ein anionisches Antirheumatikum oder ein anionisches Antibiotikum enthalten ist.

7. Wirkstoffhaltiges Pulver nach Anspruch 5 oder 6, **dadurch gekennzeichnet, daß** als anionischer Wirkstoff (a) Acamprosat, Aceclofenac, Acemetacin, Acetylcystein, Acetylsalicylsäure, Acetyltyrosin, Acipimox, Acitretin , Alanin, Alendronsäure, Amethopterin, Aminosäuren, Amoxicillin, Ampicillin , Ascorbinsäure, Atorvastatin , Azidocillin, Aztreonam, Bacampicillin, Baclofen, Benazepril, Bendamustin, Benzylpenicillin, Bezafibrat, Biotin, Bornaprin, Bumetanid, Cabastin, Canrenoinsäure, Carbamoylphenoxyessigsäure, Carbidopa, Carbimazol, Carbocistein, Carisoprodol, Cefaclor, Cefadroxil, Cefalexin, Cefazolin, Cefepim, Cefetamet, Cefixim, Cefotaxim, Cefotiam, Cefoxitin, Cefpodoxim, Ceftazidim, Ceftibuten , Ceftriaxon, Cefuroxim, Cetirizin, Chenodeoxycholsäure, Chlorambucil, Cidofovir, Cilastatin, Cilazapril, Cinoxacin, Ciprofloxacin, Cisatracurium besilat, Clavulansäure, Clodronsäure, Clorazepat, Cromoglicinsäure, Desmeninol, Diclofenac, Dicloxacillin, Enoxacin, Eprosartan, Etacrynsäure, Etidronsäure, Etofyllin, Etomidat, Felbinac, Felodipin, Fenofibrat, Fexofenadin, Flavoxat, Fleroxacin, Flucloxacillin, Flufenaminsäure, Flumazenil, Flupirtin, Flurbiprofen, Fluvastatin, Fosfomycin, Fosinopril, Furosemid, Fusidinsäure, Gabapentin, Gemfibrozil, Ibandronsäure, Ibuprofen, Iloprost, Imidapril, Imipenem, Indomethacin, Irinotecan, Isradipin, Ketoprofen, Lercanidipin , Levodopa, Levofloxacin, Liothyronin, Liponsäure, Lisinopril, Lodoxamid, Lomefloxacin, Lonazolac, Loracarbef, Loratadin, Lovastatin, Mefenaminsäure, Meropenem, Mesalazin, Metamizol, Methotrexat, Methyldopa, Mezlocillin, Moexipril, Montelukast, Moxifloxacin, Mupirocin, Naproxen, Natamycin, Nateglinid, Nedocromil, Nicotinsäure, Nifedipin, Nilvadipin, Nimodipin, Nisoldipin, Nitrendipin, Norfloxacin, Ofloxacin, Olsalazin, Orotsäure, Oxacillin, Pamidronsäure, Pangamsäure, Penicillamin, Phenoxymethylpenicillin, Pentosanpolysulfat, Perindopril, Pethidin, Pipemidsäure, Piperacillin,
Pirenoxin, Piretanid, Probenecid, Proglumid, Propicillin, Prostaglandine, Quinapril, Quinaprilat, Ramipril, Repaglinid, Reserpin, Risedronsäure, Salicylsäure, Sulfasalazin, Spirapril, Sulbactam, Sulfasalazin, Sultamicillin, Tazaroten, Tazobactam, Telmisartan, Tiagabin, Tiaprofensäure, Tilidin, Tiludronsäure, Trandolapril, Tranexamsäure, Valproinsäure Vigabatrin, Vincamin, Vinpocetin, Zanamivir, Zoledronsäure, Zopiclon und/oder deren Salze, Isomere und/oder Kombinationen enthalten sind.

8. Verwendung eines wirkstoffhaltiges Pulvers nach einem oder mehreren der Ansprüche 5 bis 7 zur Herstellung einer oralen Arzneiform mit unmittelbarem Zerfall und Wirkstofffreisetzung bereits im Mund, die nach Freisetzung für mindestens 30 Sekunden keinen oder nur einen leicht bitteren Geschmack hervorruft.

9. Verwendung des wirkstoffhaltiges Pulvers nach Anspruch 8 zur Herstellung von Arzneiformen wie verpreßten Tabletten oder Lutschtabletten, gefriergetrockneten Tabletten, gegossenen Tabletten oder Pastillen, Sachets, Kautabletten, Trockensäften, Bonbons und/oder flüssigkeitsgefüllten Bonbons.

## Claims

1. Method for producing an oral pharmaceutical form with immediate disintegration and active ingredient release even in the mouth, by vigorously mixing
(a) an anionic active pharmaceutical ingredient with
(b) a copolymer consisting of free-radical polymerized C₁ to C₄ esters of acrylic or methacrylic acid and further (meth)acrylate monomers which have functional tertiary amino groups, and
(c) 5 to 50% by weight, based on (b) , of a C₁₂ to C₂₂ carboxylic acid
in the melt, solidifying the mixture and grinding to an active ingredient-containing powder with an average particle size of 200 µm or less, incorporating the powder into a water-soluble matrix of pharmaceutically customary excipients, with the proviso that not more than 3% by weight, based on the copolymer, of emulsifiers having an HLB of at least 14 may be present.

2. Method according to Claim 1, **characterized in that** a twin-screw extruder is employed for the purpose of vigorous mixing in the melt.

3. Method according to Claim 1 or 2, **characterized in that** extrusion takes place at temperatures in the range from 80 to 200°C

4. The method as claimed in one or more of Claims 1 to 3, **characterized in that** the incorporation of the powder into the water-soluble matrix takes place by compression, casting, granulation or freeze drying.

5. Active ingredient-containing powder with an average particle size of 200 µm or less, comprising
(a) an anionic active pharmaceutical ingredient which is in the form of a solid solution and is incorporated into
(b) a copolymer which consists of free-radical polymerized C₁ to C₄ esters of acrylic or methacrylic acid and further (meth)acrylate monomers which have functional tertiary amino groups, and
(c) 5 to 50% by weight, based on (b) , of a C₁₂ to C₂₂ carboxylic acid,
(d) with the proviso that less than 3% by weight, based on the copolymer, or no emulsifier having an HLB of at least 14 is present.

6. Active ingredient-containing powder as claimed in Claim 5, **characterized in that** an anionic analgesic or an anionic antirheumatic or an anionic antibiotic is present as anionic active ingredient (a).

7. Active ingredient-containing powder as claimed in Claim 5 or 6, **characterized in that** acamprosate, aceclofenac, acemetacin, acetylcysteine, acetylsalicylic acid, acetyltyrosine, acipimox, acitretin, alanine, alendronic acid, amethopterin, amino acids, amoxicillin, ampicillin, ascorbic acid, atorvastatin, azidocillin, aztreonam, bacampicillin, baclofen, benazepril, bendamustine, benzylpenicillin, bezafibrate, biotin, bornaprine, bumetanide, cabastine, canrenoic acid, carbamoylphenoxyacetic acid, carbidopa,
carbimazole, carbocisteine, carisoprodol, cefaclor, cefadroxil, cefalexin, cefazolin, cefepime, cefetamet, cefixime, cefotaxime, cefotiam, cefoxitin, cefpodoxime, ceftazidime, ceftibuten, ceftriaxone, cefuroxime, cetirizine, chenodeoxycholic acid, chlorambucil, cidofovir, cilastatin, cilazapril, cinoxacin, ciprofloxacin, cisatracurium besilate, clavulanic acid, clodronic acid, clorazepate, cromoglicic acid, desmeninol, diclofenac, dicloxacillin, enoxacin, eprosartan, etacrynic acid, etidronic acid, etofylline, etomidate, felbinac, felodipine, fenofibrate, fexofenadine, flavoxate, fleroxacin, flucloxacillin, flufenamic acid, flumazenil, flupirtine, flurbiprofen, fluvastatin, fosfomycin, fosinopril, furosemide, fusidic acid, gabapentine, gemfibrozil, ibandronic acid, ibuprofen, iloprost, imidapril, imipenem, indomethacin, irinotecan, isradipine, ketoprofen, lercanidipine, levodopa, levofloxacin, liothyronine, lipoic acid, lisinopril, lodoxamide, lomefloxacin, lonazolac, loracarbef, loratadine, lovastatin, mefenamic acid, meropenem, mesalazine, metamizole, methotrexate, methyldopa, mezlocillin, moexipril, montelukast, moxifloxacin, mupirocin, naproxen, natamycin, nateglinide, nedocromil, nicotinic acid, nifedipine, nilvadipine, nimodipine, nisoldipine, nitrendipine, norfloxacin, ofloxacin, olsalazine, orotic acid, oxacillin, pamidronic acid, pangamic acid, penicillamine, phenoxymethylpenicillin, pentosan polysulfate, perindopril, pethidine, pipemidic acid, piperacillin, pirenoxine, piretanide, probenecid, proglumide, propicillin, prostaglandins, quinapril, quinaprilate, ramipril, repaglinide, reserpine, risedronic acid, salicylic acid, sulfasalazine, spirapril, sulbactam, sulfasalazine, sultamicillin, tazarotene, tazobactam, telmisartan, tiagabine, tiaprofenic acid, tilidine, tiludronic acid, trandolapril, tranexamic acid, valproic acid, vigabatrine, vincamine, vinpocetine, zanamivir, zoledronic acid, zopiclone and/or salts, isomers and/or combinations thereof are present as anionic active ingredient (a).

8. Use of an active ingredient-containing powder as claimed in one or more of Claims 5 to 7 for producing an oral pharmaceutical form with immediate disintegration and active ingredient release even in the mouth, which causes no or only a slight bitter taste for at least 30 seconds after release.

9. Use of the active ingredient-containing powder as claimed in Claim 8 for producing pharmaceutical forms such as compressed tablets or suckable tablets, freeze-dried tablets, cast tablets or pastilles, sachets, chewable tablets, powders for reconstitution, lozenges and/or liquid-filled lozenges.

## Revendications

1. Procédé de fabrication d'une forme pharmaceutique orale à décomposition immédiate et libération d'un principe actif dès la bouche, par mélange intensif de
(a) un principe actif pharmaceutique anionique avec
(b) un copolymère constitué d'esters en C₁ à C₄ de l'acide acrylique ou méthacrylique polymérisés par voie radicalaire et d'autres monomères de (méth)acrylate comprenant des groupes fonctionnels amino tertiaires, ainsi que
(c) 5 à 50 % en poids, par rapport à (b), d'un acide carboxylique en C₁₂ à C₂₂,
en masse fondue, solidification du mélange et broyage en une poudre contenant un principe actif ayant une taille de particule moyenne de 200 µm ou moins, incorporation de la poudre dans une matrice soluble dans l'eau constituée d'adjuvants pharmaceutiquement usuels, à condition que pas plus de 3 % en poids, par rapport au copolymère, d'émulsifiants ayant une valeur HLB d'au moins 14 puissent être contenus.

2. Procédé selon la revendication 1, **caractérisé en ce qu'**une extrudeuse bivis est utilisée pour le mélange intensif en masse fondue.

3. Procédé selon la revendication 1 ou 2, **caractérisé en ce que** l'extrusion est réalisée à des températures dans la plage allant de 80 à 200 °C.

4. Procédé selon une ou plusieurs des revendications 1 à 3, **caractérisé en ce que** l'incorporation de la poudre dans la matrice soluble dans l'eau a lieu par compression, coulée, granulation ou lyophilisation.

5. Poudre contenant un principe actif ayant une taille de particule moyenne de 200 µm ou moins, contenant :
(a) un principe actif pharmaceutique anionique, qui se présente sous la forme d'une solution solide et qui est incorporé dans
(b) un copolymère, qui est constitué d'esters en C₁ à C₄ de l'acide acrylique ou méthacrylique polymérisés par voie radicalaire et d'autres monomères de (méth)acrylate comprenant des groupes fonctionnels amino tertiaires, ainsi que
(c) 5 à 50 % en poids, par rapport à (b), d'un acide carboxylique en C₁₂ à C₂₂,
(d) à condition que pas ou moins de 3 % en poids, par rapport au copolymère, d'un émulsifiant ayant une valeur HLB d'au moins 14 soit contenu.

6. Poudre contenant un principe actif selon la revendication 5, **caractérisée en ce qu'**un analgésique anionique ou un antirhumatismal anionique ou un antibiotique anionique est contenu en tant que principe actif anionique (a).

7. Poudre contenant un principe actif selon la revendication 5 ou 6, **caractérisée en ce que** l'acamprosat, l'acéclofénac, l'acémétacine, l'acétylcystéine, l'acide acétylsalicylique, l'acétyltyrosine, l'acipimox, l'acitrétine, l'alanine, l'acide alendronique, l'améthoptérine, des acides aminés, l'amoxicilline, l'ampicilline, l'acide ascorbique, l'atorvastatine, l'azidocilline, l'aztréonam, la bacampicilline, le baclofène, le bénazépril, la bendamustine, la benzylpénicilline, le bézafibrate, la biotine, la bornaprine, le bumétanide, la cabastine, l'acide canrénoïque, l'acide carbamoylphénoxycétique, le carbidopa, le carbimazole, la carbocistéine, le carisoprodol, le céfaclor, le céfadroxil, la céfalexine, la céfazoline, le céfépim, le céfétamet, le céfixim, le céfotaxim, le céfotiam, la céfoxitine, le cefpodoxim, le ceftazidim, le ceftibutène, la ceftriaxone, le céfuroxim, la cétirizine, l'acide chénodésoxycholique, le chlorambucil, le cidofovir, la cilastatine, le cilazapril, la cinoxacine, la ciprofloxacine, le bésilate de cisatracurium, l'acide clavulanique, l'acide clodronique, le clorazépat, l'acide cromoglicique, le desméninol, le diclofénac, la dicloxacilline, l'énoxacine, l'éprosartan, l'acide étacrynique, l'acide étidronique, l'étofylline, l'étomidate, le felbinac, la félodipine, le fénofibrate, la fexofénadine, le flavoxate, la fléroxacine, la flucloxacilline, l'acide flufénamique, le flumazénil, la flupirtine, le flurbiprofène, la fluvastatine, la fosfomycine, le fosinopril, le furosémide, l'acide fusidique, la gabapentine, le gemfibrozil, l'acide ibandronique, l'ibuprofène, l'iloprost, l'imidapril, l'imipénem, l'indométhacine, l'irinotécan, l'isradipine, le kétoprofène, la lercanidipine, le lévodopa, la lévofloxacine, la liothyronine, l'acide liponique, le lisinopril, le lodoxamide, la loméfloxacine, le lonazolac, le loracarbef, la loratadine, la lovastatine, l'acide méfénamique, le méropénem, la mésalazine, le métamizol, le méthotrexate, le méthyldopa, la mezlocilline, le moexipril, le montélukast, la moxifloxacine, la mupirocine, le naproxène, la natamycine, le natéglinide, le nédocromil, l'acide nicotinique, la nifédipine, la nilvadipine, la nimodipine, la nisoldipine, la nitrendipine, la norfloxacine, l'ofloxacine, l'olsalazine, l'acide orotique, l'oxacilline, l'acide pamidronique, l'acide pangamique, la pénicillamine, la phénoxyméthylpénicilline, le polysulfate de pentosane, le périndopril, la péthidine, l'acide pipémidique, la pipéracilline, la pirénoxine, le pirétanide, le probénécide, le proglumide, la propicilline, la prostaglandine, le quinapril, le quinaprilate, le ramipril, le répaglinide, la réserpine, l'acide risédronique, l'acide salicylique, la sulfasalazine, le spirapril, le sulbactam, la sulfasalazine, la sultamicilline, le tazarotène, le tazobactam, le telmisartan, la tiagabine, l'acide tiaprofénique, la tilidine, l'acide tiludronique, le trandolapril, l'acide tranéxamique, l'acide valproïque, la vigabatrine, la vincamine, la vinpocétine, le zanamivir, l'acide zolédronique, la zopiclone et/ou leurs sels, isomères et/ou combinaisons sont contenus en tant que principe actif anionique (a).

8. Utilisation d'une poudre contenant un principe actif selon une ou plusieurs des revendications 5 à 7 pour la fabrication d'une forme pharmaceutique orale à décomposition immédiate et libération d'un principe actif dès la bouche, qui ne produit aucun goût amer ou seul un léger goût amer après libération pendant au moins 30 secondes.

9. Utilisation de la poudre contenant un principe actif selon la revendication 8 pour la fabrication de formes pharmaceutiques telles que des comprimés pressés ou des pastilles, des comprimés lyophilisés, des comprimés ou des pastilles moulés, des sachets, des comprimés à croquer, des poudres à dissoudre, des bonbons et/ou des bonbons remplis de liquide.
